# EUROPÄISCHE PATENTSCHRIFT

(11) **EP 0 567 872 B1**
(45) Veröffentlichungstag und Bekanntmachung des Hinweises auf die Patenterteilung: **06.03.1996**
(21) Anmeldenummer: 93106267.3
(22) Anmeldetag: 16.04.1993
(51) Int. Cl.: C07C 19/08, C07C 17/00

(54) **Verfahren zur Herstellung von Hexafluorbutan und dabei zugängliche Zwischenprodukte**
Process for the preparation of hexafluorbutane and accessible intermediates therein
Procédé pour la préparation d'hexafluorobutane et accessibles intermédiaires dans ce procédé

(30) Priorität: 29.04.1992 DE 4213975
(43) Veröffentlichungstag der Anmeldung: 03.11.1993
(73) Patentinhaber: BAYER AG, D-51368 Leverkusen (DE)
(72) Erfinder: Lui, Norbert, Dr., W-5000 Köln 80 (DE); Marhold, Albrecht, Dr., W-5090 Leverkusen 1 (DE); Bielefeldt, Dietmar, Dr., W-4030 Ratingen 6 (DE)

(56) Entgegenhaltungen:
- EP-A- 0 301 346
- EP-A- 0 315 783
- EP-A- 0 442 075
- EP-A- 0 442 087
- THE JOURNAL OF PHYSICAL CHEMISTRY Bd. 83, Nr. 20, 4. Oktober 1979, Seiten 2572 - 2578 T. YANO ET AL. 'Photodecomposition of 1,1-Difluoro-1,2-dichloroethane at 147 nm'

## Beschreibung

Die vorliegende Erfindung betrifft ein Verfahren zur Herstellung von Hexafluorbutan aus dem gut zugänglichen und ökologisch unbedenklichen Pentachlorbutadien und Zwischenprodukte, die durch die Umsetzung von Pentachlorbutadien mit Fluorwasserstoff zugänglich sind.

Hexafluorbutan kann als Treibmittel bei der Herstellung von Schaumstoffen eingesetzt werden und dabei die ökologisch bedenklichen Fluor-Chlor-Kohlenwasserstoffe ersetzen.

Es ist bekannt, daß man Hexafluorbutan erhalten kann, indem man Hexachlorbutadien zunächst in Gegenwart von Chlor mit Fluorwasserstoff zu Hexafluordichlorbuten umsetzt und dieses anschließend hydriert (siehe EP-A 301 346 und EP-A 442 087). Bei diesem Verfahren sind mehrere Dinge nachteilig: Hexafluordichlorbuten und das in der ersten Stufe auch gebildete Hexafluormonochlorbuten sind hochtoxische Substanzen und erfordern deshalb erhebliche Sicherheitsvorkehrungen. Das als Ausgangsprodukt benötigte Hexachlorbutadien ist wegen seiner möglichen Kanzerogenität nicht mehr technisch verfügbar und kann nur sehr aufwendig hergestellt werden Außerdem wird bei diesem Verfahren zunächst Chlor in ein Molekül eingeführt, das anschließend wieder durch Hydrierung entfernt werden muß.

Es besteht deshalb noch das Bedürfnis nach einem Verfahren zur Herstellung von Hexafluorbutan, bei dem gut zugängliche Ausgangsprodukte eingesetzt werden können und weniger toxische Zwischenstufen durchlaufen werden.

Es wurde nun ein Verfahren zur Herstellung von 1,1,1,4,4,4-Hexafluorbutan gefunden, das dadurch gekennzeichnet ist, daß man zunächst 1,1,3,4,4-Pentachlorbutadien-1,3 mit Fluorwasserstoff in Gegenwart von Katalysatoren bei 60 bis 180°C zu 1,1,1,4,4,4-Hexafluor-2-chlorbutan umsetzt und dieses in 1,1,1,4,4,4-Hexafluorbutan überführt.

Das als Ausgangsprodukt benötigte 1,1,3,4,4-Pentachlorbutadien-1,3 (im folgenden als Pentachlorbutadien bezeichnet) ist gut durch Dimerisierung von Trichlorethylen zugänglich und wenig toxisch.

In der ersten Stufe des erfindungsgemäßen Verfahrens setzt man Pentachlorbutadien mit Fluorwasserstoff in Gegenwart von Katalysatoren um. Fluorwasserstoff wird in das erfindungsgemäße Verfahren zweckmäßigerweise mindestens in der stöchiometrisch erforderlichen Menge eingesetzt, also pro Mol Pentachlorbutadien mindestens 6 Mol Fluorwasserstoff. Es ist vorteilhaft, Fluorwasserstoff im Überschuß einzusetzen, beispielsweise 6,5 bis 50 Mol pro Mol Pentachlorbutadien.

Als Katalysatoren kommen für das erfindungsgemäße Verfahren insbesondere Lewis-Säuren in Frage. Bei diesen kann es sich z.B. um Metall- und/oder Übergangsmetallhalogenide, insbesondere Metall- und/oder Übergangsmetallchloride und -fluoride handeln. Im einzelnen seien genannt: Bortrifluorid, Bortrichlorid, Antimonpentafluorid, Antimonpentachlorid, gemischte Antimon(V)-fluoride und -chloride, Titantetrachlorid, Tantalpentachlorid, Tantalpentafluorid, Niobpentachlorid, Niobpentafluorid und Zinntetrachlorid. Es können auch Gemische mehrerer Katalysatoren eingesetzt werden.

Gegebenenfalls kann man die Katalysatoren im Gemisch mit starken Sulfonsäuren einsetzen, beispielsweise im Gemisch mit Trifluormethansulfonsäure, Chlorsulfonsäure und/oder Fluorsulfonsäure. Solche Gemische können beispielsweise 0,05 bis 50 Gew.-% starke Sulfonsäuren enthalten.

Bezogen auf Pentachlorbutadien kann man beispielsweise 0,05 bis 30 Mol-% Katalysatoren einsetzen. Bevorzugt beträgt diese Menge 0,1 bis 20 Mol-%.

Die erste Stufe des erfindungsgemäßen Verfahrens wird bei Temperaturen von 60 bis 180°C durchgeführt. Vorzugsweise liegt diese Temperatur bei 100 bis 170°C.

Im allgemeinen führt man die 1. Stufe des erfindungsgemäßen Verfahrens in einem Autoklaven unter dem sich bei der Reaktionstemperatur von selbst einstellenden Druck durch. Man kann auch bei höheren oder niedrigeren Drucken arbeiten. Beispielsweise kommen Drucke in Frage, die in dem Bereich liegen, der nach unten dadurch begrenzt ist, daß der Fluorwasserstoff in flüssiger Phase vorliegt und nach oben 100 bar nicht übersteigt.

Den entstehenden Chlorwasserstoff kann man gegebenenfalls über ein Druckhalteventil entspannen.

Die Reaktionstemperatur der 1. Stufe des erfindungsgemäßen Verfahrens kann beispielsweise für 1 bis 10 Stunden aufrechterhalten werden, bevor man das Reaktionsgemisch abkühlt und aufarbeitet, beispielsweise durch Ausgießen auf Eis und/oder Wasser, Abtrennung der organischen Phase und Destillation der organischen Phase. Man kann auch so aufarbeiten, daß man unverbrauchten Fluorwasserstoff abdestilliert und anschließend das hinterbleibende Produktgemisch destillativ auftrennt.

Neben 1,1,1,4,4,4-Hexafluor-2-chlorbutan können bei der Aufarbeitung des Reaktionsgemisches der 1. Stufe des erfindungsgemäßen Verfahrens im allgemeinen eine oder mehrere Verbindungen der Formel (I) isoliert werden

CF₂X-CH₂-R (I),

in der
- X: für Fluor und
- R: für -CHCl-CF₂Cl, -CHCl-CFCl₂ oder -CCl=CCl₂ stehen.

Bei den Verbindungen der Formel (I) handelt es sich insbesondere um 1,1,4,4,4-Pentafluor-1,2-dichlorbutan und 1,1,2-Trichlor-4,4,4-trifluorbut-1-en.

Die Verbindungen der Formel (I) können in einen weiteren Ansatz zur Herstellung von 1,1,1,4,4,4-Hexafluor-2-chlorbutan aus Pentachlorbutadien und Fluorwasserstoff zurückgeführt werden und so auch zur Herstellung von 1,1,1,4,4,4-Hexafluorbutan dienen.

Gegebenenfalls im Reaktionsgemisch der Umsetzung von Pentachlorbutadien mit Fluorwasserstoff vorhandene Nebenprodukte, die noch einen geringeren Fluorierungsgrad als die Verbindungen der Formel (I) aufweisen können gemeinsam mit oder getrennt von den Verbindungen der Formel (I) in einen weiteren Ansatz zur Herstellung von 1,1,1,4,4,4-Hexafluor-2-chlor-butan aus Pentachlorbutadien und Fluorwasserstoff zurückgeführt werden.

Die Überführung des in der ersten Stufe des erfindungsgemäßen Verfahrens erhaltenen 1,1,1,4,4,4-Hexafluor-2chlorbutans in 1,1,1,4,4,4-Hexafluorbutan (im folgenden als Hexafluorbutan bezeichnet) kann mittels einer Eliminierung und einer Hydrierung erfolgen, wobei die beiden Schritte gleichzeitig oder nacheinander durchgeführt werden können.

Wenn man die Eliminierung und die Hydrierung nacheinander durchführen möchte, so kann man die Eliminierung von Chlorwasserstoff unter Bildung von 1,1,1,4,4,4-Hexafluorbut-2-en beispielsweise erreichen, indem man das 1,1,1,4,4,4-Hexafluor-2-chlorbutan mit einer wäßrigen Alkalilösung reagieren läßt. Beispielsweise kommt für diesen Zweck wäßrige Natron- oder Kalilauge im Konzentrationsbereich von 10 bis 50 Gew.-% in Frage. Man kann die Eliminierung z.B. bei Temperaturen im Bereich von -10 bis +80°C und mit überschüssiger Alkalilösung, beispielsweise mit 1 bis 5 Mol Alkali pro Mol 1,1,1,4,4,4-Hexafluor-2-chlorbutan, durchführen. Die Eliminierung kann gegebenenfalls in Gegenwart eines Lösungsvermittlers, z.B. eines Alkohols, oder eines Phasentransferkatalysators, z.B. eines quarternären Ammoniumsalzes oder eines Kronenethers, durchgeführt werden.

Die sich an die Eliminierung anschließende Hydrierung von 1,1,1,4,4,4-Hexafluorbut-2-en zu Hexafluorbutan kann in der flüssigen Phase oder in der Gasphase erfolgen. Als Katalysatoren kommen übliche Hydrierkatalysatoren in Frage, beispielsweise solche, die Palladium, Nickel oder Verbindungen davon enthalten. Die Hydrierung kann in an sich bekannter Weise vorgenommen werden, wobei in der Flüssigphase mit und ohne Lösungsmitteln und in der Gasphase mit und ohne Inertgaszusatz gearbeitet werden kann.

Es ist bevorzugt, die Eliminierung und die Hydrierung gleichzeitig durchzuführen, z.B. indem man 1,1,1,4,4,4-Hexafluor-2-chlorbutan und Wasserstoff in der Gasphase über einen geeigneten, fest angeordneten oder im Wirbel bett befindlichen Katalysator leitet. Dabei kann das Molverhältnis von 1,1,1,4,4,4-Hexafluor-2-chlorbutan zu Wasserstoff dann beispielsweise 1:0,5 bis 1:50 betragen. Vorzugsweise trägt es 1:1 bis 1:20.

Die kombinierte Eliminierung und Hydrierung kann beispielsweise bei Normaldruck oder bei erhöhten Drucken, beispielsweise im Bereich von Normaldruck bis 20 bar durchgeführt werden. Vorzugsweise wird sie bei Normaldruck durchgeführt.

Als Katalysatoren kommen dabei insbesondere solche in Frage, die Übergangsmetalle auf Trägermaterialien enthalten, Von den Übergangsmetallen sind Palladium und Platin bevorzugt, insbesondere Palladium. Die Übergangsmetalle können gegebenenfalls mit einem oder mehreren weiteren Metallen oder Metallverbindungen dotiert sein. Als Dotierungsmaterialien sind Metalle und Metallverbindungen von Elementen der 1. bis 8. Nebengruppe bevorzugt, besonders bevorzugt sind Titan, Zirkon, Nickel, Tantal und Silber und deren Verbindungen. Beispiele für Trägermaterialien sind Aktivkohlen, Aluminiumoxide, Siliciumdioxide, Bariumsulfat, Spinelle, Silikate und Titandioxid. Bevorzugt sind Aktivkohlen und Lithium-Aluminium-Spinelle. Die Katalysatoren können beispielsweise 0,5 bis 30 g Übergangsmetall pro Liter Trägermaterial enthalten Vorzugsweise liegt dieser Gehalt im Bereich von 2 bis 20 g/l.

Die Strömungsgeschwindigkeit des Reaktionsgemisches und die Katalysatormenge kann beispielsweise so gewählt werden, daß sich Katalysatorbelastungen von 10 bis 10 000 g/l x h ergeben, vorzugsweise solche von 50 bis 5000 g/l x h. Die Reaktionstemperaturen liegen im allgemeinen über 80°C, bevorzugt im Bereich 100 bis 350°C.

Es ist im allgemeinen vorteilhaft, frisch zum Einsatz gelangende Katalysatoren vor der Beaufschlagung mit 1,1,1,4,4,4-Hexafluor-2-chlorbutan zu konditionieren. Die Konditionierung kann z.B. erfolgen, indem man den Katalysator auf eine Temperatur im Bereich von 100 bis 350°C in einem Stickstoffstrom erhitzt und nach einiger Zeit ein Stickstoff/Wasserstoff-Gasgemisch über den erhitzten Katalysator leitet, wobei man den Wasserstoff-Anteil kontinuierlich oder stufenweise erhöht bis schließlich nur noch Wasserstoff über den erhitzten Katalysator geleitet wird. Man kann den Katalysator z.B. auch nur durch Erhitzen und Überleiten von Wasserstoff konditionieren.

Das nach der kombinierten Eliminierung und Hydrierung anfallende Gasgemisch kann beispielsweise aufgearbeitet werden, indem man es zunächst zur Entfernung des entstandenen Chlorwasserstoffs mit Wasser oder verdünnter Lauge wäscht und die organischen Bestandteile des Reaktionsgemisches, gegebenenfalls nach Trocknung und/oder Kühlung und/oder Phasentrennung einer Destillation unterwirft. Spuren von olefinischen Anteilen im Hexafluorbutan können gegebenenfalls durch Oxidation, beispielsweise mit Kaliumpermanganat oder Chromaten, entfernt werden.

Mit dem erfindungsgemäßen Verfahren gelingt es Hexafluorbutan unter Vermeidung von hoch toxischen Ausgangs- und Zwischenprodukten, auf wirtschaftlich vorteilhafte Weise und in guten Ausbeuten und Selektivitäten herzustellen. Dies ist ausgesprochen überraschend, denn bei der Vielzahl der möglichen Nebenreaktionen war nicht vorherzusehen, daß dies gelingen könnte. Besonders vorteilhaft ist, daß die bei der Fluorierung entstehenden, nicht vollständig fluorierten Produkte, insbesondere die Produkte der Formel (I) durch Rückführung in die Fluorierung letztendlich auch zur Herstellung von Hexafluorbutan genützt werden können.

Die folgenden Beispiele erläutern die Erfindung weiter. Soweit dort Fluorwasserstoff eingesetzt wurde handelt es sich um handelsüblichen wasserfreien Fluorwasserstoff. Die Mengenangaben bezüglich Fluorwasserstoff beziehen sich auf Messungen in der flüssigen Phase.

### Beispiele

### Beispiel 1

70 g Niobpentachlorid und 900 ml Fluorwasserstoff wurden in einem Autoklaven vorgelegt. Anschließend wurden 450 g Pentachlorbutadien zugetropft. Der Autoklav wurde verschlossen und 5 Stunden auf 140°C erhitzt. Nach dem Abkühlen auf Raumtemperatur wurde das Reaktionsgemisch auf Eis gegossen und die organische Phase abgetrennt. Die gaschromatographische Untersuchung der organischen Phase ergab, daß das Pentachlorbutadien zu 100 % umgesetzt worden war. Durch fraktionierte Destillation der organischen Phase wurden 80 g 1,1,1,4,4,4-Hexafluor-2-chlorbutan, 190 g 1,1,4,4,4-Pentafluor-1,2-dichlorbutan (Siedepunkt 83,5°C bei Normaldruck), und 80 g minder fluorierte Produkte isoliert. 1,1,4,4,4-Pentafluor-1,2-dichlorbutan und die minderfluorierten Produkte wurden dem nächsten Ansatz zugefügt.

### Beispiel 2

In einem Autoklaven wurden 350 g Niobpentachlorid vorgelegt und 950 ml Fluorwasserstoff portionsweise hinzugefügt. Anschließend wurden 81 ml Fluorsulfonsäure und 800 g Pentachlorbutadien zugetropft. Der Autoklav wurde verschlossen und 5 Stunden auf 140°C erhitzt, wobei der entstehende Chlorwasserstoff bei einem Druck von 30 bar entspannt wurde Nach dem Abkühlen auf Raumtemperatur wurde das Reaktionsgemisch auf Eis gegossen und die organische Phase abgetrennt.

Die gaschromatographische Untersuchung der organischen Phase ergab, daß das Pentachlorbutadien zu 100 % umgesetzt worden war. Durch Destillation der organischen Phase wurden 320 g 1,1,1,4,4,4-Hexafluor-2-chlorbutan und 30 g 1,1,4,4,4-Pentafluor-1,2-dichlorbutan isoliert,

¹H- und ¹⁹F-NMR-Daten von 1,1,4,4,4-Pentafluor-1,2-dichlorbutan: ¹H-NMR δ = 2,5-3,1 (m, 2H, CH₂), 4,43 (dd, 1H, CH); ¹⁹F NMR δ = 61 (dd, CF₂Cl), 64,7 (t, CF₃).

### Beispiel 3

In einem Autoklaven wurden 150 ml Fluorwasserstoff vorgelegt und 50 g Tantalpentachlorid hinzugefügt. Anschließend wurden bei 0°C 100 g Pentachlorbutadien zugetropft. Die Reaktionsmischung wurde dann 5 Stunden auf 140°C erhitzt, wobei der entstehende Chlorwasserstoff bei einem Druck von 30 bar entspannt wurde. Nach dem Abkühlen auf Raumtemperatur wurde das Reaktionsgemisch auf Eis gegossen und die organische Phase abgetrennt. Durch Destillation der organischen Phase wurden 30 g 1,1,1,4,4,4-Hexafluor-2-chlorbutan isoliert.

### Beispiel 4

In einem Autoklaven wurden 960 ml Fluorwasserstoff vorgelegt und 80 g Antimonpentachlorid hinzugefügt. Anschließend wurden bei 0°C 450 g Pentachlorbutadien zugetropft. Die Reaktionsmischung wurde dann 5 Stunden auf 150°C erhitzt, wobei der entstehende Chlorwasserstoff bei einem Druck von 40 bar entspannt wurde. Nach dem Abkühlen auf Raumtemperatur wurde das Reaktionsgemisch auf Eis gegossen und die organische Phase abgetrennt. Durch Destillation der organischen Phase wurden 149 g 1,1,1,4,4,4-Hexafluor-2-chlorbutan, 157 g 1,1,4,4,4-Pentafluor-1,2-dichlorbutan und 21 g minder fluorierte Produkte isoliert.

### Beispiel 5

In einem Autoklaven wurden 40 g Antimonpentachlorid vorgelegt und 480 ml Fluorwasserstoff portionsweise hinzugefügt. Anschließend wurden 11 ml Fluorsulfonsäure und 220 g Pentachlorbutadien zugetropft. Der Autoklav wurde verschlossen und 5 Stunden auf 140°C erhitzt. Nach dem Abkühlen auf Raumtemperatur wurde das Reaktionsgemisch auf Eis gegossen und die organische Phase abgetrennt. Die gaschromatographische Untersuchung der organischen Phase ergab, daß das Pentachlorbutadien zu 100 % umgesetzt worden war. Als Produkte wurden durch Destillation 63 g 1,1,1,4,4,4-Hexafluor-2-chlorbutan und 57 g 1,1,4,4,4-Pentafluoro-1,2-dichlorobutan isoliert.

### Beispiel 6

In einem Autoklaven wurden 480 ml Fluorwasserstoff vorgelegt und 40 g Antimonpentachlorid hinzugefügt. Anschließend wurde der Autoklav verschlossen und die Mischung 5 Stunden auf 60°C erhitzt. Nach Abkühlen auf Raumtemperatur wurden 230 g Pentachlorbutadien zugetropft. Die Reaktionsmischung wurde dann für 5 Stunden auf 140°C erhitzt. Nach dem Abkühlen auf Raumtemperatur wurde das Reaktionsgemisch auf Eis gegossen und die organische Phase abgetrennt. Durch Destillation wurden 50 g 1,1,1,4,4,4-Hexafluoro-2-chlorobutan, 97 g 1,1,4,4,4-Pentafluor-1,2-dichlorbutan und 14 g minder fluorierte Produkte isoliert.

### Beispiel 7

In einem Autoklaven wurden 18 g Antimonpentachlorid vorgelegt und 480 ml Fluorwasserstoff portionsweise hinzugefügt. Anschließend wurden 220 g Pentachlorbutadien zugetropft. Der Autoklav wurde verschlossen und 4 Stunden auf 120°C erhitzt. Nach dem Abkühlen auf Raumtemperatur wurde das Reaktionsgemisch auf Eis gegossen und die organische Phase abgetrennt. Die gaschromatographische Untersuchung der organischen Phase ergab, daß das Pentachlorbutadien zu 100 % umgesetzt worden war. Durch Destillation wurden 14 g 1,1,4,4,4-Pentafluor-1,2-dichlorbutan, 10 g 1,1,2-Trichlor-1,4,4,4-tetrafluorbutan, 75 g 1,1,2-Trichlor-4,4,4-trifluorbut-1-en (Siedepunkt 126°C bei Normaldruck) und 10 g minder fluorierte Produkte isoliert.

¹H- und ¹⁹F-NMR-Daten von 1,1,2-Trichloro-4,4,4-trifluorobut-1-en: ¹H-NMR δ = 3,42 (q, J = 9,4 Hz, 2H, CH₂); ¹⁹F-NMR δ = 64,73 (t, J = 9,4 Hz, CF₃).

¹H- und ¹⁹F-NMR-Daten von 1,1,2-Trichloro-1,4,4,4-tetrafluorbutan: ¹H NMR δ = 2,72 (m, 1H, CH₂), 3,06 (m, 1H, CH₂), 4,58 (m, 1H, CHCl); ¹⁹F NMR δ = 59,39 (d, J = 4,3 Hz, CFCl₂), 64,54 (t, J = 9,7 Hz, CF₃).

### Beispiel 8

60 g Titantetrachlorid und 1000 ml Fluorwasserstoff wurden in einem Autoklaven vorgelegt. Anschließend wurden 500 g Pentachlorbutadien zugetropft. Der Autoklav wurde verschlossen und 7 Stunden auf 150°C erhitzt. Nach dem Abkühlen auf Raumtemperatur wurde das Reaktionsgemisch auf Eis gegossen und die organische Phase abgetrennt. Durch Destillation der organischen Phase wurden 10 g 1,1,1,4,4,4-Hexafluor-2-chlorbutan, 190 g 1,1,4,4,4-Pentafluor-1,2-dichlorbutan, 60 g 1,1,2-Trichlor-4,4,4-trifluorbut-1-en und 90 g minder fluorierte Produkte isoliert.

### Beispiel 9

52 g Zinntetrachlorid, 900 ml Fluorwasserstoff und 450 g Pentachlorbutadien wurden in einen Autoklaven eingebracht und anschließend 5 Stunden auf 140°C erhitzt. Nach dem Abkühlen auf Raumtemperatur wurde das Reaktionsgemisch auf Eis gegossen und die organische Phase abgetrennt. Durch Destillation der organischen Phase wurden 230 g 1,1,2-Trichlor-4,4,4-trifluorbut-1-en und 139 g 1,1,2,4-Tetrachlor-4,4-difluorbuten-1 (Siedepunkt 45,5-46°C/12 mbar) isoliert.

¹H- und ¹⁹F-NMR-Daten von 1,1,2,4-Tetrachlor-4,4-difluorbuten-1: ¹H-NMR δ = 3,64 (t, J = 12 Hz, CH₂); ¹⁹F-NMR δ = 50,28 (t, J = 12 Hz, CF₂Cl).

### Beispiel 10

20 g Bortrifluorid, 480 ml Fluorwasserstoff und 230 g Pentachlorbutadien wurden in einen Autoklaven eingebracht und anschließend 5 Stunden auf 150°C erhitzt. Nach dem Abkühlen auf Raumtemperatur wurde das Reaktionsgemisch auf Eis gegossen und die organische Phase abgetrennt. Nach Destillation der organischen Phase wurden 80 g 1,1,2,4-Tetrachlor-4,4-difluorbuten-1, 9 g 1,1,2-Trichlor-4,4,4-trifluorbuten-1, 60 g Pentachlorbutadien und 22 g minder fluorierte Produkte isoliert.

### Beispiel 11

In einem Autoklaven wurden 10 g Antimonpentafluorid vorgelegt und 200 ml Fluorwasserstoff portionsweise hinzugefügt. Anschließend wurden 110 g Pentachlorbutadien zugetropft. Der Autoklav wurde verschlossen und 5 Stunden auf 140°C erhitzt. Nach dem Abkühlen auf Raumtemperatur wurde das Reaktionsgemisch auf Eis gegossen und die organische Phase abgetrennt. Die gaschromatographische Untersuchung der organischen Phase ergab, daß das Pentachlorbutadien zu 100 % umgesetzt worden war. Durch Destillation der organischen Phase wurden 19 g 1,1,1,4,4,4-Hexafluor-2-chlorbutan, 55 g 1,1,4,4,4-Pentafluoro-1,2-dichlorobutan und 11 g minder fluorierte Produkte isoliert.

### Beispiele 12 und 13

Ein senkrecht angeordneter, elektrisch beheizbarer Rohrreaktor aus Quarz (Länge 310 mm, Durchmesser 36 mm) wurde mit 200 ml eines Trägerkatalysators beschickt, der pro Liter eines kugelförmigen Lithium-Aluminium-Spinells (Kugeldurchmesser 3 bis 5 mm) 18 g Palladium enthielt. Der Katalysator wurde 6 Stunden lang bei 200°C unter hindurchleiten von 20 bis 25 ml Wasserstoff pro Stunde konditioniert. Danach wurden die beiden unten beschriebenen Hydrierungen durchgeführt. Die das Quarzrohr verlassenden Gase wurden bei -78°C kondensiert und mit ¹⁹F-NMR- und GC-MS-Kopplungs-Spektroskopie untersucht.

### Beispiel 12

Einsätze: 0,06 Mol 1,1,1,4,4,4-Hexafluor-2-chlorbutan und 0,3 Mol Wasserstoff.

Reaktionsbedingungen: 200°C Normaldruck

Katalysatorbelastung: 300 g/l x h

Hexafluorbutan wurde mit einem Umsatz von 90 % und einer Selektivität von 92 % erhalten.

### Beispiel 13

Einsätze: 0,15 Mol 1,1,1,4,4,4-Hexafluor-2-chlorbutan und 0,9 Mol Wasserstoff
Reaktionsbedingungen: 300°C Normaldruck
Katalysatorbelastung: 300 g/l x h
Hexafluorbutan wurde mit einem Umsatz von 92 % und in einer Selektivität von 83 % erhalten.

### Beispiel 14

Es wurde in der gleichen Apparatur gearbeitet wie in den Beispielen 12 und 13, jedoch wurde als Katalysator kugelförmige Aktivkohle eingesetzt, die pro Liter 5 g Palladium enthielt. Die Konditionierung des Katalysators erfolgte in der Weise, daß er auf 235°C erhitzt und zunächst 1 Stunde lang mit einem Stickstoffstrom von 100 ml/h beaufschlagt wurde. Danach wurden jeweils 30 Minuten lang Stickstoff/Wasserstoff-Gemische in einer Menge von jeweils 100 ml/h über den Katalysator geleitet, wobei der Wasserstoffgehalt der Gemische in vier Stufen von 4:1 über 3:2 und 2:3 auf 1:4 Volumenteile erhöht wurde. Abschließend wurden im Verlauf von 30 Minuten 50 ml Wasserstoff bei 235°C über den Katalysator geleitet.

Dann wurde die erfindungsgemäße kombinierte Eliminierung und Hydrierung wie folgt durchgeführt.
Einsätze: 1,5 Mol 1,1,1,4,4,4-Hexafluor-2-chlorbutan und 4,0 Mol Wasserstoff
Reaktionsbedingungen: 300°C, Normaldruck
Katalysatorbelastung 300 g/l x h
Hexafluorbutan wurde mit einem Umsatz von größer als 99,5 % und einer Selektivität von größer als 99 % erhalten.

### Beispiel 15

Es wurde verfahren wie in Beispiel 14, jedoch wurde der Katalysator nicht konditioniert.
Einsätze: 0,2 Mol 1,1,1,4,4,4-Hexafluor-2-chlorbutan und 2,5 Mol Wasserstoff
Reaktionsbedingungen: 250°C, Normaldruck
Katalysatorbelastung 200 g/l x h
Hexafluorbutan wurde mit einem Umsatz von 83 % und einer Selektivität von 94,8 % erhalten.

## Patentansprüche

1. Verfahren zur Herstellung von 1,1,1,4,4,4-Hexafluorbutan, dadurch gekennzeichnet, daß man zunächst 1,1,3,4,4-Pentachlorbutadien-1,3 mit Fluorwasserstoff in Gegenwart von Katalysatoren bei 60 bis 180°C zu 1,1,1,4,4,4-Hexafluor-2-chlorbutan umsetzt und dieses in 1,1,1,4,4,4-Hexafluorbutan überführt.

2. Verfahren nach Anspruch 1, dadurch gekennzeichnet, daß man in der ersten Stufe pro Mol Pentachlorbutadien mindestens 6 Mol Fluorwasserstoff einsetzt.

3. Verfahren nach Ansprüchen 1 und 2, dadurch gekennzeichnet, daß man in der ersten Stufe Lewis-Säuren als Katalysatoren, gegebenenfalls im Gemisch mit starken Sulfonsäuren, einsetzt.

4. Verfahren nach Ansprüchen 1 bis 3, dadurch gekennzeichnet, daß man das in der ersten Stufe erhaltene 1,1,1,4,4,4-Hexafluor-2-chlorbutan mittels einer Eliminierung und einer Hydrierung, die gegebenenfalls gleichzeitig erfolgen können, in 1,1,1,4,4,4-Hexafluorbutan überführt.

5. Verfahren nach Ansprüchen 1 bis 4, dadurch gekennzeichnet, daß man die Eliminierung und die Hydrierung nacheinander durchführt, wobei man die Eliminierung unter Bildung von 1,1,1,4,4,4-Hexafluorbut-2-en durch reagieren lassen mit einer wäßrigen Alkalilösung und dessen Hydrierung katalytisch in der flüssigen Phase oder in der Gasphase durchführt.

6. Verfahren nach Anspruch 4, dadurch gekennzeichnet, daß man die Eliminierung und die Hydrierung gleichzeitig durchführt, indem man 1,1,1,4,4,4-Hexafluor-2-chlorbutan und Wasserstoff in der Gasphase über einen fest angeordneten oder im Wirbelbett befindlichen Katalysator leitet.

7. Verfahren nach Ansprüchen 1 bis 6, dadurch gekennzeichnet, daß man als Katalysatoren solche verwendet, die Übergangsmetalle auf Trägermaterialien enthalten.

8. Verfahren nach Ansprüchen 5 und 6, dadurch gekennzeichnet, daß die Reaktionstemperatur über 80°C beträgt.

9. Verbindungen der Formel (I)
CF₂X-CH₂-R (I),
in der
X für Fluor und
R für -CHCl-CF₂Cl, -CHCl-CFCl₂ oder -CCl=CCl₂ stehen.

10. Verbindungen nach Anspruch 9, dadurch gekennzeichnet, daß es sich um 1,1,4,4,4-Pentafluor-1,2-dichlorbutan oder 1,1,2-Trichlor-4,4,4-trifluorbut-1-en handelt.

## Claims

1. Process for the preparation of 1,1,1,4,4,4- hexafluorobutane, characterised in that 1,1,3,4,4-pentachlorobuta-1,3-diene is first reacted with hydrogen fluoride in the presence of catalysts at 60 to 180°C to give 1,1,1,4,4,4-hexafluoro-2-chlorobutane, and this is converted to 1,1,1,4,4,4-hexafluorobutane.

2. Process according to Claim 1, characterised in that at least 6 mol of hydrogen fluoride are used per mol of pentachlorobutadiene in the first stage.

3. Process according to Claims 1 and 2, characterised in that Lewis acids are used as catalysts in the first stage, optionally in a mixture with strong sulphonic acids.

4. Process according to Claims 1 to 3, characterised in that the 1,1,1,4,4,4-hexafluoro-2-chlorobutane obtained in the first stage is converted to 1,1,1,4,4,4-hexafluorobutane by means of elimination and hydrogenation, which can optionally be effected simultaneously.

5. Process according to Claims 1 to 4, characterised in that the elimination and the hydrogenation are carried out successively, the elimination to form 1,1,1,4,4,4-hexafluorobut-2-ene being carried out by reaction with an aqueous alkali solution and catalytic hydrogenation thereof in the liquid phase or in the gas phase.

6. Process according to Claim 4, characterised in that the elimination and the hydrogenation are carried out simultaneously by passing 1,1,1,4,4,4-hexafluoro-2-chlorobutane and hydrogen in the gas phase over a catalyst in a fixed or fluidised bed.

7. Process according to Claims 1 to 6, characterised in that the catalysts used are those containing transition metals on support materials.

8. Process according to Claims 5 and 6, characterised in that the reaction temperature is above 80°C.

9. Compounds of formula (I):
CF₂X-CH₂-R (I)
in which
X is fluorine and
R is -CHCl-CF₂Cl, -CHCl-CFCl₂ or -CCl=CCl₂.

10. Compounds according to Claim 9, characterised in that they are 1,1,4,4,4-pentafluoro-1,2-dichlorobutane or 1,1,2-trichloro-4,4,4-trifluorobut-1-ene.

## Revendications

1. Procédé de préparation du 1,1,1,4,4,4-hexafluorobutane caractérisé en ce que l'on convertit d'abord le 1,1,3,4,4-pentachlorobutadiène-1,3, à l'aide du fluorure d'hydrogène et en présence de catalyseurs à des températures de 60 à 180°C, en le 1,1,1,4,4,4-hexafluoro-2-chlorobutane qu'on convertit ensuite en le 1,1,1,4,4,4-hexafluorobutane.

2. Procédé selon la revendication 1, caractérisé en ce que, dans le premier stade opératoire, on met en oeuvre au moins 10 mol de fluorure d'hydrogène par mole du pentachlorobutadiène.

3. Procédé selon les revendications 1 et 2, caractérisé en ce que, au premier stade, on utilise en tant que catalyseurs des acides de Lewis, éventuellement en mélange avec des acides sulfoniques forts.

4. Procédé selon les revendications 1 à 3, caractérisé en ce que le 1,1,1,4,4,4-hexafluoro-2-chlorobutane obtenu au premier stade est converti en le 1,1,1,4,4,4-hexafluorobutane par une élimination et une hydrogénation qui le cas échéant peuvent être réalisées simultanément.

5. Procédé selon les revendications 1 à 4, caractérisé en ce que l'on procède à l'élimination et à l'hydrogénation successivement, l'élimination avec formation du 1,1,1,4,4,4-hexafluorobut-2-ène étant réalisée par réaction avec une lessive alcaline et l'hydrogénation par hydrogénation catalytique en phase liquide ou en phase gazeuse.

6. Procédé selon la revendication 4, caractérisé en ce que l'on réalise simultanément l'élimination et l'hydrogénation en envoyant le 1,1,1,4,4,4-hexafluoro-2-chlorobutane et l'hydrogène en phase gazeuse sur un catalyseur en lit fixe ou en lit tourbillonnaire.

7. Procédé selon les revendications 1 à 6, caractérisé en ce que l'on utilise des catalyseurs contenant des métaux de transition sur des matières de support.

8. Procédé selon les revendications 5 et 6, caractérisé en ce que la température de réaction est supérieure à 80°C.

9. Composés de formule (I):
CF₂X-CH₂-R (I),
dans laquelle
X représente le fluor et
R représente -CHCl-CF₂Cl, -CHCl-CFCl₂ ou -CCl=CCl₂.

10. Composés selon la revendication 9, caractérisés en ce qu'ils consistent en le 1,1,4,4,4-pentafluoro-1,2-dichlorobutane ou le 1,1,2-trichloro-4,4,4-trifluorobut-1-ène.
